(19) Europäisches Patentamt
European Patent Office
Office européen des brevets

(11) Veröffentlichungsnummer: **0 169 515 B1**

# (12) EUROPÄISCHE PATENTSCHRIFT

(45) Veröffentlichungstag der Patentschrift: **11.10.89**

(51) Int. Cl.⁴: **C 07 J 1/00**, A 61 K 31/565// C07J51/00, C07B59/00

(21) Anmeldenummer: **85109074.6**

(22) Anmeldetag: **19.07.85**

## (54) Neue 17alpha-Halogenvinyl-Estran-Derivate, ihre Herstellung und Verwendung in der Medizin.

(30) Priorität: **25.07.84 DE 3427795**

(43) Veröffentlichungstag der Anmeldung: **29.01.86 Patentblatt 86/05**

(45) Bekanntmachung des Hinweises auf die Patenterteilung: **11.10.89 Patentblatt 89/41**

(84) Benannte Vertragsstaaten: **AT BE CH DE FR GB IT LI LU NL SE**

(56) Entgegenhaltungen:
**EP-A- 0 137 434**
**US-A- 4 450 149**
**THE JOURNAL OF NUCLEAR MEDICINE, Band 23, Nr. 5, Mai 1982, Seiten 431-436, New York, US; R.N. HANSON et al.: "E-17alpha[125I]iodovinylestradiol: An estrogen-receptor-seeking radiopharmaceutical"**
**JOURNAL OF NUCLEAR MEDICINE, Band 25, Nr. 4, April 1984, Seiten 472-477, New York, US; E.M. JAGODA et al.: "[I-125] 17alpha-iodovinyl 11beta-methoxyestradiol: In vivo and in vitro properties of a high-affinity estrogen-receptor radiopharmaceutical"**
**CHEMICAL ABSTRACTS, Band 100, Nr. 7, 13. Februar** 1984, Seite 536, Nr. 51042f, Columbus, Ohio, US; G.W. KABALKA et al.: "Synthesis of vinyl bromides via reaction of vinyl boronic acids with sodium bromide", & SYNTH. COMMUN. 1983, 13(12), 1027-32
CHEMICAL ABSTRACTS, 10th Collective Index, Band 86-95, 1977-1981, Chemical Substances, Seite 35091CS und Chemical Abstracts, Band 95, Nr. 7, 17. August 1981, Seite 649, Nr. 61355c, Columbus, Ohio, US; G.W. KABALKA et al.: "A convenient procedure for the syntheses of vinyl iodides via the reaction of iodine monochloride with vinylboronic acids", & SYNTH. COMMUN. 1981, 11(3), 247-51
SYNTHETIC COMMUNICATIONS, Band 13(1983), Seiten 1027-1032

(73) Patentinhaber: **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder: **Hofmeister, Helmut, Dr.**
**Weislingenstrasse 4**
**D-1000 Berlin 28 (DE)**
Erfinder: **Laurent, Henry, Dr.**
**Glambecker Weg 21**
**D-1000 Berlin 28 (DE)**
Erfinder: **Schulze, Paul-Eberhard, Dr.**
**Endestrasse 30**
**D-1000 Berlin 39 (DE)**
Erfinder: **Wiechert, Rudolf, Prof. Dr.**
**Petzower Strasse 8A**
**D-1000 Berlin 39 (DE)**
Erfinder: **Elger, Walter, Dr.**
**Schorlemmer Allee 12 B**
**D-1000 Berlin 33 (DE)**
Erfinder: **Pollow, Kunhard, Prof. Dr.**
**Lion Feuchtwangerstrasse 63**
**D-6500 Mainz-Hechtsheim (DE)**
Erfinder: **Grill, Hans-Jörg**
**Neue Universitätsstrasse 5**
**D-6500 Mainz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft 17α-Brom- und 17α-Iodvinyl-estran-Derivate der allgemeinen Formel I

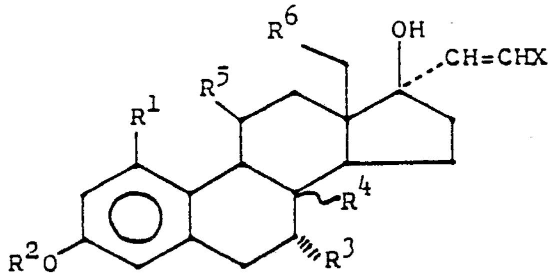

(I)

worin
X ein Brom- oder ein Jodatom in Z-Stellung,
$R^1$ Wasserstoff oder Hydroxy,
$R^2$ Wasserstoff oder Alkyl mit bis zu 3 C-Atomen,
$R^3$ Wasserstoff oder Methyl,
$R^4$ ein α- oder β-ständiges Wasserstoffatom,
$R^5$ Wasserstoff, Methyl oder Methoxy und
$R^6$ Wasserstoff oder Methyl darstellen,
mit der Maßgabe, daß $R^1$, $R^3$, $R^5$ und $R^6$ nicht Wasserstoff sind und $R^2$ nicht Methyl ist, wenn X Brom bedeutet.

Die Verbindungen der Formel I sind pharmakologisch wirksame Substanzen mit einem Wirkprofil wie die entsprechenden 17α-Ethinylverbindungen, wie z. B. das bekannte Estrogen Ethinylestradiol.

In Form ihrer radioaktiv markierten Derivate sind die Verbindungen der Formel I auch wertvolle Diagnostika.

Aus der US-Patentschrift 4,450,149 sowie aus den Arbeiten von Hanson et al. (J. Nucl. Med. 23 (1982) 431) ist in der E-konfigurierten Estrogen-Reihe das 17α-(E-2-Jodvinyl)-1.3.5(10)-estratrien-3.17β-diol bekannt geworden. Auch der entsprechende 3-Methylether (Kabalka et al., Syn. Commun. 11 (1981) 247), die entsprechende Radio-Jod-Verbindung (Kabalka et al., J. Label Comp. Radiopharm. 19 (1982) 795) und die 11β-Methoxy-Verbindung, nämlich das 17α-(E-2-Jodvinyl)-11β-methoxy-1.3.5(10)-estratrien-3.17β-diol (Jagoda et al., J. Nucl. Med. 25 (1984) 472) sind bereits hergestellt worden.

In der Z-konfigurierten Estrogen-Reihe ist das 17α-(Z-2-Bromvinyl)-3-methoxy-1.3.5(10)-estratrien-17β-ol bekannt (Kabalka et al., Syn. Commun. 13 (1983) 1027).

Die Synthese dieser Halogenvinyl-Steroide sowie auch die der Radiojodvinyl-Verbindungen verläuft über die 17α-(E-2-Tributylstannylvinyl)-Derivate oder über die 17α-(E-2-Vinylboran)-Verbindung, die mit Jod oder [$^{125}$I] Jod umgesetzt werden. Die 17α-Tributylstannyle werden aus Ethinylestradiol oder dessen 3-Methylether, z. B. durch Umsetzung mit Tributylzinnhydrid unter Zugabe eines Radikalbildners wie z. B. α.α'-Azoisobutyronitril in Tetrahydrofuran hergestellt, während das E-17α-Vinylboran durch Hydroborierung mit Organoboranen wie z. B. Catecholboran entsteht.

Zu der bekannten 17α-(Z-2-Bromvinyl)-Verbindung gelangt man über das entsprechende 17α-(E-2-Vinylboran)-Steroid durch Reaktion mit Natriumbromid und N-Chlorsuccinimid.

Die Verbindungen der Formel I werden bevorzugt nach einem neuen Verfahren hergestellt, welches dadurch gekennzeichnet, daß man ein 17α-Ethinylestran-Derivat der allgemeinen Formel II

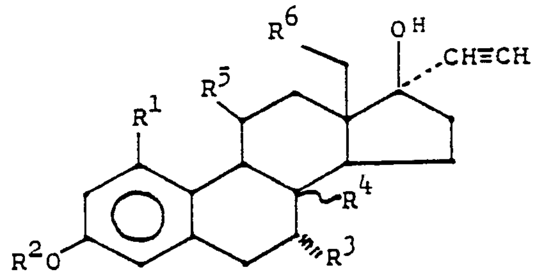

(II)

worin $R^{1-6}$ die oben angegebene Bedeutung haben, in einem polaren Lösungsmittel mit einem Trialkylzinnhydrid mit 2-6 C-Atomen pro Alkylrest bei Temperaturen von 20-100 °C behandelt und anschließend das so erhaltene 17α-Trialkylstannylvinyl-estran mit einer positives Halogen abgebenden Verbindung umsetzt.

Hierzu wird das Ausgangsmaterial der Formel II in einem inerten polaren oder unpolaren Lösungsmittel wie z. B. Tetrahydrofuran, 2-Dimethoxyethan, Acetonitril, Hexamethylphosphorsäuretriamid, Dioxan, i-Propanol und N-Methylpyrrolidon mit einem Trialkyl- oder Triphenylzinnhydrid bei Temperaturen von 20-100 °C behandelt. Ein Radikalbildner als Katalysator wird wie in der Reihe der E-konfigurierten Verbindungen nicht zugesetzt. Die Reaktionszeit beträgt in Abhängigkeit von den Reaktionsbedingungen wenige Stunden bei bis mehrere Tage und ist nach längstens 4 Tagen beendet.

Das Triphenyl- oder Trialkylzinnhydrid wird im Überschuß angewendet. Die eingesetzte Menge beträgt das 2 bis 10fache bezogen auf die Menge des Steroids.

Der Verlauf der Reaktion war durchaus überraschend, da allein das Weglassen des Radikalbildners bei der an sich bekannten Reaktion zur Herstellung von E-konfigurierten Verbindungen zu Verbindungen führt, die Z-konfiguriert sind.

Der sich anschließende Austausch der Trialkylstannylgruppe gegen Brom oder Jod erfolgt stereospezifisch wie in der E-konfigurierten Reihe.

Die 17α-(2-Bromvinyl)-Steroide der allgemeinen Formel I können Ausgangsprodukte für die Herstellung der entsprechenden 17α-(2-Radio-jodvinyl)-Verbindungen sein. So lassen sich z. B. aus 17α-(2-Bromvinyl)-Steroide durch Austauschreaktion mit radioaktivem Natrium-[*I]-jodid die entsprechenden 17α-(2-[*I]-Jodvinyl)-Verbindungen herstellen. Eine weitere Methode zur Herstellung von 17α-(Z-2-[*I]-Jodvinyl)- bzw. 17α-(Z-2-[*Br]-Bromvinyl)-Steroiden verläuft über die 17α-(Z-2-Tributylstannylvinyl)-Verbindungen, die mit Na($^{125}$I) jodid bzw. Na($^{77}$Br)-bromid umgesetzt werden.

Geeignete radioaktive Iodisotope sind beispielsweise das $^{124}$I-, $^{125}$I-, $^{126}$I- oder $^{131}$I-Isotop. Geeignete radioaktive Bromoisotope sind beispielsweise das $^{77}$Br- und $^{82}$Br-Isotop.

Die 17α-Jodvinylestradiole der allgemeinen Formel I besitzen eine starke estrogene Wirkung und eine wesentlich stärkere Bindung am Estrogenrezeptor als die isomeren E-17α-Halogenvinylestradiole. So ist die Affinität zum Estrogenrezeptor beim 17α-(Z-2-Jodvinyl)-1.3.5(10)-estratrien-3.17β-diol 3mal stärker als beim Ethinylestradiol, während die isomere E-2-Jodvinyl-Verbindung gegenüber Ethinylestradiol eine wesentlich schwächere Rezeptorbindung aufweist (0.6x Ethinylestradiol).

Die Verbindungen der Formel I mit radioaktivem Halogen sind aufgrund ihrer starken Rezeptorbindung als Radiodiagnostika geeignet. Mit Hilfe der Szintigraphie lassen sie sich somit zur Darstellung von Organen und Tumoren, die Estrogen-Rezeptoren enthalten, welche von den Vinylhalogen-Steroiden besetzt werden, verwenden. Bei den Organen handelt es sich bei der Frau insbesondere um den Uterus und die Brustdrüsen. Weiterhin können die Radiohalogenvinyl-Steroide zur Auffindung von Tumoren und Metastasen in den Geweben der aufgeführten Organe eingesetzt werden. Von besonderem Interesse sind die 17α-[$^{125}$-I]-Jodvinyl-Verbindungen, da sie sich für in-vitro Diagnostika eignen.

Die nachfolgenden Ausführungsbeispiele dienen zur näheren Erläuterung der Erfindung.

## Beispiel 1

17α-(Z-2-Iodvinyl)-1.3.5(10)-estratrien-3.17β-diol

a) 10,0 g 3-Benzoyloxy-17α-ethinyl-1.3.5(10)-estratrien-17β-ol in 200 ml Tetrahydrofuran werden bei 70 °C mit 30 ml Tributylzinnhydrid gerührt. Nach 4 Tagen destilliert man das Lösungsmittel ab und chromatographiert den Rückstand an Kieselgel mit Toluol/Essigester. Es werden 3,5 g 3-Benzoyloxy-17α-(Z-2-tributylstannylvinyl)-1.3.5(10)-estratrien-17β-ol als Öl isoliert.

760 mg 3-Benzoyloxy-17α-(Z-2-tributylstannylvinyl)-1.3.5(10)estratrien-17β-ol werden in 15 ml Tetrahydrofuran mit 300 mg N-Iodsuccinimid bei Raumtemperatur gerührt. Nach 45 min verdünnt man mit Essigester, wäscht mit Wasser, trocknet und engt im Vakuum ein. Das Rohprodukt wird in 10 ml Methanol und 1,2 ml Wasser mit 120 mg Natriumcarbonat bei Raumtemperatur gerührt. Man gibt nach 1 Stunde Essigester zu, wäscht mit Wasser und trocknet. Das Rohprodukt wird an Kieselgel mit Hexan/Essigester chromatographiert. Es werden 320 mg 17α-(Z-2-Iodvinyl)-1.3.5(10)-estratrien-3.17β-diol erhalten.

Schmelzpunkt : 95 °C (Zersetzung).

b) 10,0 g 3.17β-Diacetoxy-17α-ethinyl-1.3.5(10)-estratrien werden in 150 ml N-Methylpyrrolidon und 80 ml Tributylzinnhydrid bei 70 °C gerührt. Nach 48 Stunden wird mit Essigester verdünnt und mehrmals mit Wasser gewaschen, getrocknet und im Vakuum weitgehend eingeengt. Nach Chromatographieren des Rohproduktes an Kieselgel mit Toluol/Essigester werden 2,9 g 17β-Acetoxy-17α-(Z-2-tributylstannylvinyl)-1.3.5(10)-estratrien-3-ol als Öl erhalten.

510 mg 17β-Acetoxy-17α-(Z-2-tributylstannylvinyl)-1.3.5(10)-estratrien-3-ol werden bei Raumtemperatur mit 250 mg N-Iodsuccinimid in 15 ml Tetrahydrofuran gerührt. Nach 30 min verdünnt man mit Essigester, wäscht mit Wasser, trocknet und engt im Vakuum ein. Das Rohprodukt wird in 8 ml Methanol und 1 ml Wasser mit 100 mg Natriumcarbonat bei Raumtemperatur gerührt. Nach 1 Stunde wird mit Essigester verdünnt, mit Wasser gewaschen, getrocknet und im Vakuum eingeengt. Das Rohprodukt wird an Kieselgel mit Hexan/Essigester chromatographiert. Man erhält 240 mg 17α-(Z-2-Iodvinyl)-1.3.5(10)-estratrien-3.17β-diol.

Schmelzpunkt : 97 °C (Zersetzung).

Beispiel 2

17α-(Z-2-Bromvinyl)-1.3.5(10)-estratrien-3.17β-diol

630 mg 3-Benzoyloxy-17α-(Z-2-tributylstannylvinyl)-1.3.5(10)-estratrien-17β-ol werden in 12 ml Tetrahydrofuran mit 280 mg N-Bromsuccinimid bei Raumtemperatur gerührt. Das Reaktionsgemisch wird nach 30 min mit Essigester verdünnt, mit Wasser gewaschen, getrocknet und im Vakuum eingeengt. Den Rückstand löst man in einem Gemisch aus 8 ml Methanol und 1 ml Wasser und rührt mit 100 mg Natriumcarbonat bei Raumtemperatur. Das Reaktionsgemisch wird in Essigester aufgenommen, mit Wasser gewaschen und getrocknet. Nach Chromatographieren des Rohproduktes mit Hexan/Essigester erhält man 270 mg 17α-(Z-2-Bromvinyl)-1.3.5(10)-estratrien-3.17β-diol.

Schmelzpunkt : 93 °C (Zersetzung).

Beispiel 3

17α-(Z-2-Iodvinyl)-3-methoxy-1.3.5(10)-estratrien-17β-ol

8,0 g 17α-Ethinyl-3-methoxy-1.3.5(10)-estratrien-17β-ol werden analog Beispiel 1 mit Tributylzinnhydrid umgesetzt. Es werden 2,1 g 3-Methoxy-17α-(Z-2-tributylstannylvinyl)-1.3.5(10)-estratrien-17β-ol als Öl erhalten.

900 mg 3-Methoxy-17α-(Z-2-tributylstannylvinyl)-1.3.5(10)-estratrien-17β-ol werden in 15 ml Tetrahydrofuran und 400 mg N-Iodsuccinimid bei Raumtemperatur umgesetzt. Nach 45 min verdünnt man mit Essigester, wäscht mit Wasser und trocknet. Das Rohprodukt wird mit Hexan/Essigester chromatographiert. Es werden 470 mg 17α-(2-Iodvinyl)-3-methoxy-1.3.5(10)-estratrien-17β-ol als Schaum isoliert.

Beispiel 4

17α-(Z-2-Iodvinyl)-7α-methyl-1.3.5(10)-estratrien-3.17β-diol

3,9 g 17α-Ethinyl-7α-methyl-1.3.5(10)-estratrien-3.17β-diol (DE-AS-1 443 683 (1969)) werden in 40 ml N-Methylpyrrolidon mit ,40 ml Tributylzinnhydrid bei 70 °C umgesetzt. Das Reaktionsgemisch wird nach 48 Stunden mit Essigester verdünnt, mehrfach mit Wasser gewaschen, getrocknet und im Vakuum weitgehend eingeengt. Nach Chromatographieren des Rohproduktes mit Toluol/Essigester erhält man 1,4 g 7α-Methyl-17α-(Z-2-tributylstannylvinyl)-1.3.5(10)-estratrien-3.17β-diol als Öl.

620 mg 7α-Methyl-17α-(Z-2-tributylstannylvinyl)-1.3.5(10)-estratrien-3.17β-diol werden analog Beispiel 3 mit N-Iod-succinimid umgesetzt. Es werden 340 mg 17α-(Z-2-Iodvinyl)-7α-methyl-1.3.5(10)-estratrien-3.17β-diol erhalten.

Beispiel 5

17α-(Z-2-Bromvinyl)-7α-methyl-1.3.5(10)-estratrien-3.17β-diol

420 mg 7α-Methyl-17α-(Z-2-tributylstannylvinyl)-1.3.5(10)-estratrien-3.17β-diol werden in 10 ml Tetrahydrofuran mit 260 mg N-Bromsuccinimid bei Raumtemperatur umgesetzt. Nach 30 min verdünnt man mit Essigester, wäscht mit Wasser und trocknet. Nach Chromatographieren an Kieselgel mit Hexan/Essigester erhält man 210 mg 17α-(Z-2-Bromvinyl)-7α-methyl-1.3.5(10)-estratrien-3.17β-diol als Schaum.

Beispiel 6

17α-(Z-2-Iodvinyl)-1.3.5(10)-estratrien-1.3.17β-triol

5,6 g 1.3-Diacetoxy-17α-ethinyl-1.3.5(10)-estratrien-17β-ol (US-PS-3 418 415 (1968)) werden analog Beispiel 4 mit Tributylzinnhydrid umgesetzt. Es werden 1,6 g 17α-(Z-2-Tributylstannylvinyl)-1.3.5(10)-estratrien-1.3.17β-triol als Öl erhalten.

380 mg 17α-(Z-2-Tributylstannylvinyl)-1.3.5(10)-estratrien-1.3.17β-triol werden analog Beispiel 3 mit N-Iodsuccinimid umgesetzt. Man erhält 130 mg 17α-(Z-2-Iodvinyl)-1.3.5(10)-estratrien-1.3.17β-triol.

Beispiel 7

17α-(Z-2-Bromvinyl)-1.3.5(10)-estratrien-1.3.17β-triol

510 mg 17α-(Z-2-Tributylstannylvinyl)-1.3.5(10)-estratrien-1.3.17β-triol werden analog Beispiel 5 mit N-Bromsuccinimid umgesetzt. Ausbeute 240 mg 17α-(Z-2-Bromvinyl)-1.3.5(10)-estratrien-1.3.17β-triol.

Beispiel 8

17α-(Z-2-Iodvinyl)-18-methyl-1.3.5(10)-estratrien-3.17β-diol

4,0 g 17α-Ethinyl-18-methyl-1.3.5(10)-estratrien-3.17β-diol (US-PS-3 519 714 (1970)) werden analog Beispiel 4 mit Tributylzinnhydrid umgesetzt. Es werden 1,8 g 18-Methyl-17α-(Z-2-tributylstannylvinyl)-1.3.5(10)-estratrien-3.17β-diol als Öl isoliert.

800 mg 18-Methyl-17α-(Z-2-tributylstannylvinyl)-1.3.5(10)-estratrien-3.17β-diol werden analog Beispiel 3 mit N-Iodsuccinimid umgesetzt. Man erhält 380 mg 17α-(Z-2-Iodvinyl)-18-methyl-1.3.5(10)-estratrien-3.17β-diol.

Beispiel 9

17α-(Z-2-Bromvinyl)-18-methyl-1.3.5(10)-estratrien-3.17β-diol

650 mg 18-Methyl-17α-(Z-2-tributylstannylvinyl)-1.3.5(10)-estratrien-3.17β-diol werden analog Beispiel 5 mit N-Bromsuccinimid umgesetzt. Man erhält 310 mg 17α-(Z-2-Bromvinyl)-18-mlethyl-1.3.5(10)-estratrien-3.17β-diol.

Beispiel 10

17α-(Z-2-Iodvinyl)-8α-estra-1.3.5(10)-trien-3.17β-diol

5,0 g 17α-Ethinyl-8α-estra-1.3.5(10)-trien-3.17β-diol (US-PS-3 407 217 (1968)) werden analog Beispiel 4 mit Tributylzinnhydrid umgesetzt. Es werden 2,2 g 17α-(Z-2-Tributylstannylvinyl)-8α-estra-1.3.5(10)-trien-3.17β-diol als Öl erhalten.

2,1 g 17α-(Z-2-Tributylstannylvinyl)-8α-estra-1.3.5(10)-trien-3.17β-diol werden analog Beispiel 3 mit N-Iodsuccinimid umgesetzt. Man erhält 980 mg 17α-(Z-2-Iodvinyl)-8α-estra-1.3.5(10)-trien-3.17β-diol.

Beispiel 11

17α-(Z-2-Bromvinyl)-8α-estra-1.3.5(10)-trien-3.17β-diol

1,3 g 17α-(Z-2-Tributylstannylvinyl)-8α-estra-1.3.5(10)-trien-3.17β-diol werden analog Beispiel 5 umgesetzt. Es werden 610 mg 17α-(Z-2-Bromvinyl)-8α-estra-1.3.5(10)-trien-3.17β-diol erhalten.

Beispiel 12

17α-(Z-2-Iodvinyl)-11β-methoxy-1.3.5(10)-estratrien-3.17β-diol

4,0 g 17α-Ethinyl-11β-methoxy-1.3.5(10)-estratrien-3.17β-diol (US-PS-3 579 545 (1971)) werden analog Beispiel 4 mit Tributylzinnhydrid umgesetzt. Es werden 1,8 g 11β-Methoxy-17α-(Z-2-tributylstannylvinyl)-1.3.5(10)-estratrien-3.17β-diol als Öl isoliert.

850 mg 11β-Methoxy-17α-(Z-2-tributylstannylvinyl)-1.3.5(10)-estratrien—3.17β-diol werden analog Beispiel 3 mit N-Iodsuccinimid umgesetzt. Man erhällt 380 mg 17α-(Z-2-Iodvinyl)-11β-methoxy-1.3.5(10)-estratrien-3.17β-diol.

Beispiel 13

17α-(Z-2-Bromvinyl)-11β-methoxy-1.3.5(10)-estratrien-3.17β-diol

350 mg 11β-Methoxy-17α-(Z-2-tributylstannylvinyl)-1.3.5(10)-estratrien-3.17β-diol werden analog Beispiel 5 umgesetzt. Es werden 150 mg 17α-(Z-2-Bromvinyl)-11β-methoxy-1.3.5(10)-estratrien-3.17β-diol erhalten.

Beispiel 14

17α-(Z-2-Iodvinyl)-11β-methyl-1.3.5(10)-estratrien-3.17β-diol

2,3 g 17α-Ethinyl-11β-methyl-1.3.5(10)-estratrien-3.17β-diol (DE-AS-1 593 444 (1976)) werden analog Beispiel 4 mit Tributylzinnhydrid umgesetzt. Es werden 900 mg 11β-Methyl-17α-(Z-2-tributylstannylvinyl)-1.3.5(10)-estratrien-3.17β-diol als Öl erhalten.

350 mg 11β-Methyl-17α-(Z-2-tributylstannylvinyl)-1.3.5(10)-estratrien-3.17β-diol werden analog Beispiel 3 mit N-Iodsuccinimid umgesetzt. Man erhält 160 mg 17α-(Z-2-Iodvinyl)-11β-methyl-1.3.5(10)-estratrien-3.17β-diol.

Beispiel 15

17α-(Z-2-Bromvinyl)-11β-methyl-1.3.5(10)-estratrien-3.17β-diol

300 mg 11β-Methyl-17α-(Z-2-tributylstannylvinyl)-1.3.5(10)-estratrien-3.17β-diol werden analog Beispiel 5 mit N-Bromsuccinimid umgesetzt. Es werden 110 mg 17α-(Z-2-Bromvinyl)-11β-methyl-1.3.5(10)-estratrien-3.17β-diol isoliert.

Beispiel 16

Zu 500 μg 17α-(Z-2-Tributylstannylvinyl)-1.3.5(10)-estratrien-3.17β-diol in 250 μl Methylethylketon werden bei Zimmertemperatur 50 μl Na-$^{125}$I, enthaltend 185 MBq$^{125}$I, als wässrig neutrale Lösung zugegeben. Man läßt 5 min bei Zimmertemperatur stehen, trennt die entstandenen Stoffe auf einer analytischen HPTLC-Platte und eluiert die radioaktive Zone. Das Eluat wird über eine halbpräparative HPLC-Säule mit Radioaktivitätsmonitor aufgetrennt. Man erhält > 148 MBq reines 17α-(Z-2-$^{125}$-Iodvinyl)-1.3.5(10)-estratrien-3.17β-diol in methanolisch wässriger Lösung. Die radioaktive Markierung ist trägerfrei. Die spezifische Aktivität entspricht der des eingesetztten radioaktiven Iods.

Analog wie für $^{125}$Iod beschrieben, erhält man die Verbindung auch mit $^{123}$Iod, $^{131}$Iod und $^{132}$Iod markiert, ebenso mit $^{77}$Brom, $^{80m}$Brom und $^{82}$Brom.

Die wässrige Phase wird 2 mal mit Chloroform gewaschen, die organischen Phasen werden vereinigt, mit Magnesiumsulfat getrocknet, sulfat getrocknet, filtriert und eingeengt. Man trennt auf analytischen HPTLC-Platten, eluiert den radioaktiven Streifen mit Ethanol und erhält ~ 37 MBq [$^{125}$I] trägerfrei markiertes 17α-(Z-2-Iodvinyl)-1.3.5(10)-estratrien-3.17β-diol.

## Patentansprüche

1. 17α-Brom- und 17α-Iodvinyl-estran-Derivate der allgemeinen Formel I

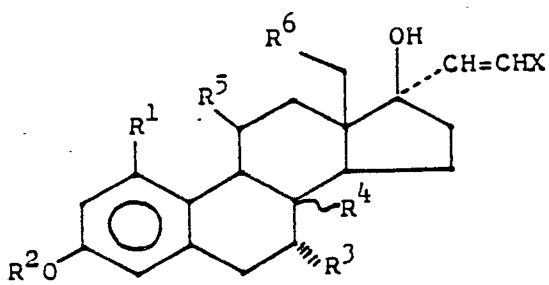

(I)

worin

X ein Brom- oder ein Jodatom in Z-Stellung,
$R^1$ Wasserstoff oder Hydroxy,
$R^2$ Wasserstoff oder Alkyl mit bis zu 3 C-Atomen,
$R^3$ Wasserstoff oder Methyl,
$R^4$ ein α- oder β-ständiges Wasserstoffatom,
$R^5$ Wasserstoff, Methyl oder Methoxy und
$R^6$ Wasserstoff oder Methyl darstellen,

mit der Maßgabe, daß $R^1$, $R^3$, $R^5$ und $R^6$ nicht Wasserstoff sind und $R^2$ nicht Methyl ist, wenn X Brom bedeutet.

2. 17α-(Z-2-Iodvinyl)-1.3.5(10)-estratrien-3.17β-diol.
3. 17α-(Z-2-Bromvinyl)-1.3.5(10)-estratrien-3.17β-diol.
4. 17α-(Z-2-Iodvinyl)-3-methoxy-1.3.5(10)-estratrien-17β-ol.
5. 17α-(Z-2-Iodvinyl)-7α-methyl-1.3.5(10)-estratrien-3.17β-diol.
6. 17α-(Z-2-Bromvinyl)-7α-methyl-1.3.5(10)-estratrien-3.17β-diol.
7. 17α-(Z-2-Iodvinyl)-1.3.5(10)-estratrien-1.3.17β-triol.
8. 17α-(Z-2-Bromvinyl)-1.3.5(10)-estratrien-1.3.17β-triol.
9. 17α-(Z-2-Iodvinyl)-18-methyl-1.3.5(10)-estratrien-3.17β-diol.
10. 17α-(Z-2-Bromvinyl)-18-methyl-1.3.5(10)-estratrien-3.17β-diol.
11. 17α-(Z-2-Iodvinyl)-8α-estra-1.3.5(10)-trien-3.17β-diol.
12. 17α-(Z-2-Bromvinyl)-8α-estra-1.3.5(10)-trien-3.17β-diol.
13. 17α-(Z-2-Iodvinyl)-11β-methoxy-1.3.5(10)-estratrien-3.17β-diol.
14. 17α-(Z-2-Bromvinyl)-11β-methoxy-1.3.5(10)-estratrien-3.17β-diol.
15. 17α-(Z-2-Iodvinyl)-11β-methyl-1.3.5(10)-estratrien-3.17β-diol.
16. 17α-(Z-2-Bromvinyl)-11β-methyl-1.3.5(10)-estratrien-3.17β-diol.

17. 17α-(Z-2-[$^{125}$I]-Iodvinyl)-1.3.5(10)-estratrien-3.17β-diol.

18. 17α-Brom- und 17α-Jodvinyl-estran-Derivate gemäß den Ansprüchen 1-16, dadurch gekennzeichnet, daß sie ein radioaktives Bromisotop oder Jodisotop enthalten.

19. Diagnostisches Mittel, dadurch gekennzeichnet, daß es ein radioaktiv markiertes 17α-Halovinylestran-Derivat gemäß Anspruch 17 und 18 enthält.

20. Verfahren zur Herstellung von 17α-Halovinylestran-Derivaten der Formel I

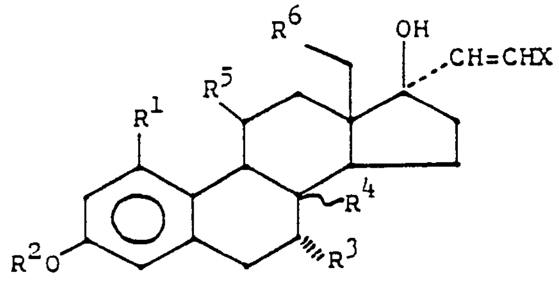

(I)

worin

X ein Brom- oder ein Jodatom in Z-Stellung
$R^1$ Wasserstoff oder Hydroxy,
$R^2$ Wasserstoff oder Alkyl mit bis zu 3 C-Atomen
$R^3$ Wasserstoff oder Methyl,
$R^4$ ein α- oder β-ständiges Wasserstoffatom,
$R^5$ Wasserstoff, Methyl oder Methoxy und
$R^6$ Wasserstoff oder Methyl darstellen,

dadurch gekennzeichnet, daß man ein 17α-Ethinyl-estran-Derivat der allgemeinen Formel II

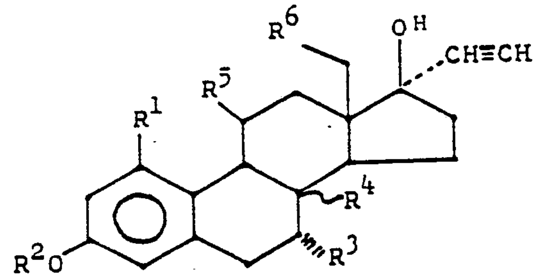

(II)

worin $R^{1-6}$ die oben angegebene Bedeutung haben, in einem polaren Lösungsmittel mit einem Trialkylzinnhydrid mit 2-6 C-Atomen pro Alkylrest bei Temperaturen von 20-100 °C behandelt und anschließend das so erhaltene 17α-Trialkylstannylvinyl)-estran mit einer positives Halogen abgebenden Verbindung umsetzt.

**Claims**

1. 17α-bromo- and 17α-iodo-vinyloestrane derivatives of the general formula I

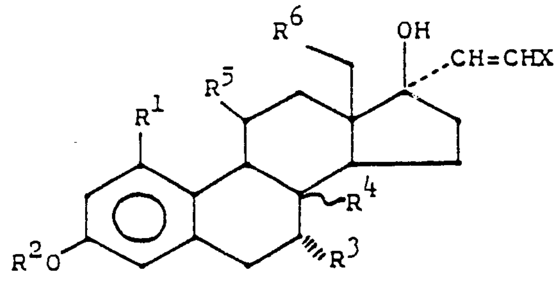

(I)

wherein

X represents a bromine or iodine atom in the Z-configuration,
$R^1$ represents hydrogen or hydroxy,
$R^2$ represents hydrogen or alkyl having up to 3 carbon atoms,

$R^3$ represents hydrogen or methyl,
$R^4$ represents a hydrogen atom in the α- or β-configuration,
$R^5$ represents hydrogen, methyl or methoxy and
$R^6$ represents hydrogen or methyl,
with the proviso that $R^1$, $R^3$, $R^5$ and $R^6$ are not hydrogen and $R^2$ is not methyl when X represents bromine.

2. 17α-(Z-2-iodovinyl)-1,3,5(10)-oestratriene-3,17β-diol.
3. 17α-(Z-2-bromovinyl)-1,3,5(10)-oestratriene-3,17β-diol.
4. 17α-(Z-2-iodovinyl)-3-methoxy-1,3,5(10)-oestratrien-17β-ol.
5. 17α-(Z-2-iodovinyl)-7α-methyl-1,3,5(10)-oestratriene-3,17β-diol.
6. 17α-(Z-2-bromovinyl)-7α-methyl-1,3,5(10)-oestratriene-3,17β-diol.
7. 17α-(Z-2-iodovinyl)-1,3,5(10)-oestratriene-1,3,17β-triol.
8. 17α-(Z-2-bromovinyl)-1,3,5(10)-oestratriene-1,3,17β-triol.
9. 17α-(Z-2-iodovinyl)-18-methyl-1,3,5(10)-oestratriene-3,17β-diol.
10. 17α-(Z-2-bromovinyl)-18-methyl-1,3,5(10)-oestratriene-3,17β-diol.
11. 17α-(Z-2-iodovinyl)-8α-oestra-1,3,5(10)-triene-3,17β-diol.
12. 17α-(Z-2-bromovinyl)-8α-oestra-1,3,5(10)-triene-3,17β-diol.
13. 17α-(Z-2-iodovinyl)-11β-methoxy-1,3,5(10)-oestratriene-3,17β-diol.
14. 17α-(Z-2-bromovinyl)-11β-methoxy-1,3,5(10)-oestratriene-3,17β-diol.
15. 17α-(Z-2-iodovinyl)-11β-methyl-1,3,5(10)-oestratriene-3,17β-diol.
16. 17α-(Z-2-bromovinyl)-11β-methyl-1,3,5(10)-oestratriene-3,17β-diol.
17. 17α-(Z-2-[$^{125}$I]-iodovinyl)-1,3,5(10)-oestratriene-3,17β-diol.
18. 17α-bromo- and 17α-iodo-vinyloestrane derivatives according to claims 1 to 16, characterised in that they contain a radioactive bromine isotope or iodine isotope.
19. Diagnostic agent, characterised in that it contains a radioactively labelled 17α-halovinyloestrane derivative according to claims 17 and 18.
20. Process for the preparation of 17α-halovinyloestrane derivatives of formula I

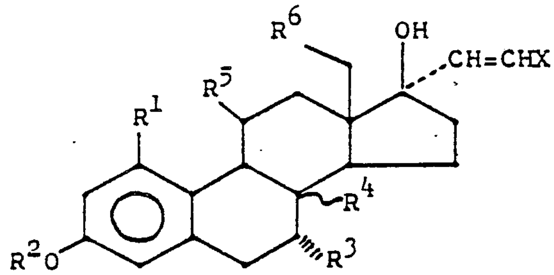

(I)

wherein
X represents a bromine or iodine atom in the Z-configuration,
$R^1$ represents hydrogen or hydroxy,
$R^2$ represents hydrogen or alkyl having up to 3 carbon atoms,
$R^3$ represents hydrogen or methyl,
$R^4$ represents a hydrogen atom in the α- or β-configuration,
$R^5$ represents hydrogen, methyl or methoxy and
$R^6$ represents hydrogen or methyl,
characterised in that a 17α-ethynyloestrane derivative of the general formula II

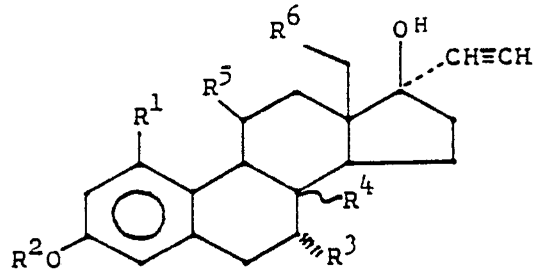

(II)

wherein $R^{1-6}$ have the meanings given above, is treated at temperatures of from 20 to 100 °C in a polar solvent with a trialkyltin hydride having from 2 to 6 carbon atoms per alkyl radical, and the 17α-(trialkylstannylvinyl)-oestrane so obtained is then reacted with a compound yielding positive halogen.

## Revendications

1. Bromovinyl-17α estranes et iodo-vinyl-17α oestranes répondant à la formule générale I :

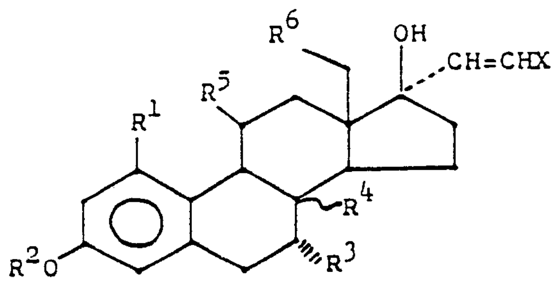

(I)

dans laquelle :

X représente un atome de brome ou d'iode ayant la configuration Z,
$R^1$ représente l'hydrogène ou un hydroxy,
$R^2$ représente l'hydrogène ou un alkyle ayant au plus 3 atomes de carbone,
$R^3$ représente l'hydrogène ou un méthyle,
$R^4$ représente un atome d'hydrogène ayant la configuration α ou β,
$R^5$ représente l'hydrogène, un méthyle ou un méthoxy, et
$R^6$ représente l'hydrogène ou un méthyle,

avec la condition que $R^1$, $R^3$, $R^5$ et $R^6$ ne représentent pas l'hydrogène et que $R^2$ ne représente pas un méthyle dans le cas où X représente le brome.

2. (Iodo-2 vinyl-Z)-17α estratriène-1,3,5(10) diol-3,17β.
3. (Bromo-2 vinyl-Z)-17α estratriène-1,3,5(10) diol-3,17β.
4. (Iodo-2 vinyl-Z)-17α méthoxy-3 estratriène-1,3,5(10) ol-17β.
5. (Iodo-2 vinyl-Z)-17α méthyl-7α estratriène-1,3,5(10) diol-3,17β.
6. (Bromo-2 vinyl-Z)-17α méthyl-7α estratriène-1,3,5(10) diol-3,17β.
7. (Iodo-2 vinyl-Z)-17α estratriène-1,3,5(10) triol-1,3,17β.
8. (Bromo-2 vinyl-Z)-17α estratriène-1,3,5(10) triol-1,3,17β.
9. (Iodo-2 vinyl-Z)-17α méthyl-18 estratriène-1,3,5(10) diol-3,17β.
10. (Bromo-2 vinyl-Z)-17α méthyl-18 estratriène-1,3,5(10) diol-3,17β.
11. (Iodo-2 vinyl-Z)-17α 8α-estratriène-1,3,5(10) diol-3,17β.
12. (Bromo-2 vinyl-Z)-17α 8α-estratriène-1,3,5(10) diol-3,17β.
13. (Iodo-2 vinyl-Z)-17α méthoxy-11β estratriène-1,3,5(10) diol-3,17β.
14. (Bromo-2 vinyl-Z)-17α méthoxy-11β estratriène-1,3,5(10) diol-3,17β.
15. (Iodo-2 vinyl-Z)-17α méthyl-11β estratriène-1,3,5(10) diol-3,17β.
16. (Bromo-2 vinyl-Z)-17α méthyl-11β estratriène-1,3,5(10) diol-3,17β.
17. (Iodo-[$^{125}$I]-2 vinyl-Z)-17α estratriène-1,3,5(10) diol-3,17β.
18. Bromovinyl-17α estranes et iodovinyl-17α estranes selon l'une quelconque des revendications 1 à 16, caractérisés en ce qu'ils contiennent un isotope radio-actif du brome ou de l'iode.
19. Produit de diagnostic caractérisé en ce qu'il contient un halogénovinyl-17α estrane marqué par un élément radio-actif selon l'une des revendications 17 et 18.
20. Procédé de préparation d'halogénovinyl-17α estranes répondant à la formule I :

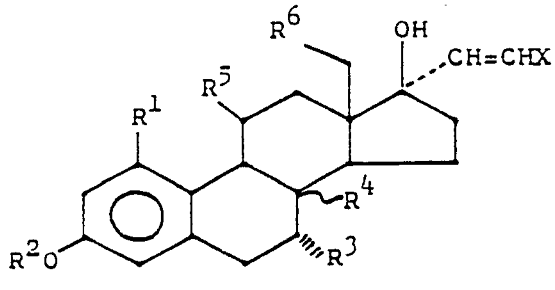

(I)

dans laquelle

X représente un atome de brome ou d'iode ayant la configuration Z,
$R^1$ représente l'hydrogène ou un hydroxy,
$R^2$ représente l'hydrogène ou un alkyle contenant au plus 3 atomes de carbone,
$R^3$ représente l'hydrogène ou un méthyle,
$R^4$ représente un atome d'hydrogène ayant la configuration α ou la configuration β,
$R^5$ représente l'hydrogène, un méthyle ou un méthoxy, et

$R^6$ représente l'hydrogène ou un méthyle,
procédé caractérisé en ce qu'on traite à des températures de 20 à 100 °C un éthynyl-17α estrane répondant à la formule générale II :

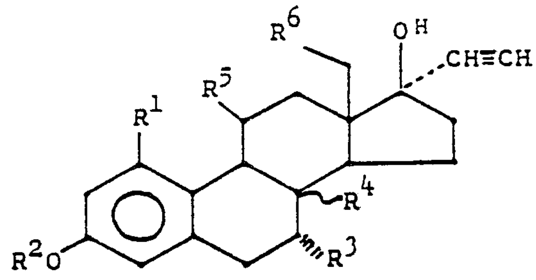

(II)

dans laquelle $R^1$ à $R^6$ ont les significations qui leur ont été données ci-dessus, dans un solvant polaire, par un hydrure de trialkyl-étain dont chacun des alkyles contient de 2 à 6 atomes de carbone, puis on fait réagir le (trialkylstannyl-vinyl)-17α estrane ainsi obtenu avec un composé capable de céder un halogène positif.